(19) 

Europäisches Patentamt
European Patent Office
Office européen des brevets

(11) **EP 4 174 165 A1**

(12) **EUROPEAN PATENT APPLICATION**
published in accordance with Art. 153(4) EPC

(43) Date of publication:
**03.05.2023 Bulletin 2023/18**

(21) Application number: **21829581.4**

(22) Date of filing: **24.05.2021**

(51) International Patent Classification (IPC):
$C12M\ 1/34$ (2006.01)     $G01B\ 9/021$ (2006.01)
$G01N\ 37/00$ (2006.01)

(52) Cooperative Patent Classification (CPC):
**C12M 1/34; G01B 9/021; G01N 37/00**

(86) International application number:
**PCT/JP2021/019618**

(87) International publication number:
**WO 2021/261148 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.06.2020 JP 2020109804**

(71) Applicant: **FUJIFILM Corporation
Tokyo 106-8620 (JP)**

(72) Inventor: **YAMAMOTO, Hiroaki
Ashigarakami-gun, Kanagawa 258-8577 (JP)**

(74) Representative: **Klunker IP
Patentanwälte PartG mbB
Destouchesstraße 68
80796 München (DE)**

(54) **IMAGING SYSTEM AND IMAGING DEVICE**

(57)     Provided are an imaging system and an imaging device capable of generating a super-resolution interference fringe image of an object to be observed flowing through a flow channel. A light source that irradiates light in a first direction and irradiates light toward a flow channel through which an object to be observed flows in a second direction orthogonal to the first direction, an imaging sensor that has an imaging surface orthogonal to the first direction and on which a plurality of pixels are two-dimensionally arranged in a manner non-parallel to the second direction and that images light passing through the flow channel to output an interference fringe image, and an information processing device that generates a super-resolution interference fringe image based on a plurality of interference fringe images output from the imaging sensor are included.

FIG. 3

EP 4 174 165 A1

**Description**

Technical Field

The technique of the present disclosure relates to an imaging system and an imaging device.

Background Art

In order to reduce a size of a device that images a small object to be observed, such as a cell, so-called lens-free digital holography in which an optical-system component is eliminated is known. In the digital holography, the object to be observed is imaged by using a light source that emits coherent light such as a laser beam and an imaging sensor, and an interference fringe image obtained by the imaging is reconstructed to generate a reconstructed image.

Further, it is known to apply a super-resolution technique to this digital holography (refer to, for example, JP2014-507645A). The super-resolution technique is a technique for generating an image having a resolution exceeding a resolution of the imaging sensor. JP2014-507645A discloses that an interference fringe image having super-resolution (hereinafter referred to as super-resolution interference fringe image) is generated based on a plurality of images obtained by irradiating an object to be observed with light from a plurality of irradiation positions having different irradiation angles. With reconstruction of the super-resolution interference fringe image, a high-definition reconstructed image can be obtained.

In digital holography, it is known that imaging is performed on a cell or the like flowing through a microchannel (also referred to as a microfluidic channel), which is a minute flow channel, as an observation target (refer to, for example, JP2017-075958A). JP2017-075958A discloses that an interference fringe image is processed in real time while capturing a moving image of an object to be observed flowing through the microchannel by an imaging sensor.

SUMMARY OF THE INVENTION

Technical Problem

In the technique described in JP2014-507645A, it is necessary to perform a plurality of times of imaging by irradiating the object to be observed with light from the plurality of irradiation positions. Thus, the object to be observed is assumed to be stationary during the plurality of times of imaging. For this reason, in the technique described in JP2014-507645A, it is difficult to generate a super-resolution interference fringe image of the object to be observed flowing through the microchannel as described in JP2017-075958A.

An object of the technique of the present disclosure is to provide an imaging system and an imaging device capable of generating a super-resolution interference fringe image of an object to be observed flowing through a flow channel.

Solution to Problem

In order to achieve the above object, an imaging system of the present disclosure comprises a light source that irradiates light in a first direction and irradiates light toward a flow channel through which an object to be observed flows in a second direction orthogonal to the first direction, an imaging sensor that has an imaging surface orthogonal to the first direction and on which a plurality of pixels are two-dimensionally arranged in a manner non-parallel to the second direction and that images light passing through the flow channel to output an interference fringe image, and an information processing device that generates a super-resolution interference fringe image based on a plurality of interference fringe images output from the imaging sensor.

It is preferable that the plurality of pixels are arranged in the X direction at a first arrangement pitch and arranged in the Y direction orthogonal to the X direction at a second arrangement pitch in the imaging surface.

It is preferable that a diagonal direction vector with the first arrangement pitch as the X-direction component and the second arrangement pitch as the Y-direction component is parallel to the second direction.

It is preferable that the first arrangement pitch is equal to the second arrangement pitch.

It is preferable that as for a deviation amount of two interference fringe images output from the imaging sensor in two consecutive imaging cycles, a component in the X direction is a non-integer multiple of the first arrangement pitch and a component in the Y direction is a non-integer multiple of the second arrangement pitch.

It is preferable that as for the deviation amount, the component in the X direction is smaller than the first arrangement pitch and the component in the Y direction is smaller than the second arrangement pitch.

It is preferable that the information processing device calculates the deviation amount based on the two interference fringe images output from the imaging sensor in the two consecutive imaging cycles and generates the super-resolution interference fringe image based on the calculated deviation amount and the two interference fringe images.

It is preferable that the information processing device reconstructs the super-resolution interference fringe image to generate a reconstructed image.

The information processing device executes reconstruction processing of generating the reconstructed image while changing a reconstruction position, in-focus position detection processing of calculating sharpness of the reconstructed image each time the reconstructed image is generated by the reconstruction processing and detecting an in-focus position where the calculated sharpness is maximized, and optimal reconstructed image output processing of outputting the reconstructed image at the in-focus position detected by the in-focus position detection processing as an optimal reconstructed image.

A light source that irradiates light in a first direction and irradiates light toward a flow channel through which an object to be observed flows in a second direction orthogonal to the first direction, and an imaging sensor that has an imaging surface orthogonal to the first direction and on which a plurality of pixels are two-dimensionally arranged in a manner non-parallel to the second direction and that images light passing through the flow channel to output an interference fringe image.

Advantageous Effects of Invention

According to the technique of the present disclosure, it is possible to provide an imaging system and an imaging device capable of generating the super-resolution interference fringe image of the object to be observed flowing through the flow channel.

BRIEF DESCRIPTION OF THE DRAWINGS

Fig. 1 is a diagram showing an example of a configuration of a digital holography system.
Fig. 2 is a diagram showing an example of a configuration of an imaging device.
Fig. 3 is a plan view of an example of a positional relationship between a microchannel and an imaging sensor.
Fig. 4 is a diagram showing an example of a pixel arrangement of the imaging sensor.
Fig. 5 is a diagram for describing a diagonal direction vector.
Fig. 6 is a diagram showing a state in which an interference fringe is generated by a cell.
Fig. 7 is a diagram showing a wavefront in a case where diffracted light and transmitted light strengthen each other.
Fig. 8 is a diagram showing a wavefront in a case where diffracted light and transmitted light weaken each other.
Fig. 9 is a diagram showing an example of an interference fringe image output from the imaging sensor.
Fig. 10 is a block diagram showing an example of a hardware configuration of an information processing device.
Fig. 11 is a block diagram showing an example of a functional configuration of the information processing device.
Fig. 12 is a diagram for describing an imaging operation of the imaging device.
Fig. 13 is a diagram schematically showing an example of deviation amount calculation processing.
Fig. 14 is a diagram showing an example of a relationship between a first arrangement pitch and a second arrangement pitch of an X-direction component and a Y-direction component of a deviation amount D.
Fig. 15 is a diagram schematically showing an example of registration processing and integration processing.
Fig. 16 is a diagram for describing reconstruction processing.
Fig. 17 is a flowchart showing an example of a flow of repetition processing.
Fig. 18 is a diagram showing an example of processing of searching for an in-focus position.
Fig. 19 is a diagram showing a modification example of the imaging sensor.
Fig. 20 is a diagram for describing a diagonal direction vector according to the modification example.

DESCRIPTION OF EMBODIMENTS

An example of an embodiment according to the technique of the present disclosure will be described with reference to accompanying drawings.

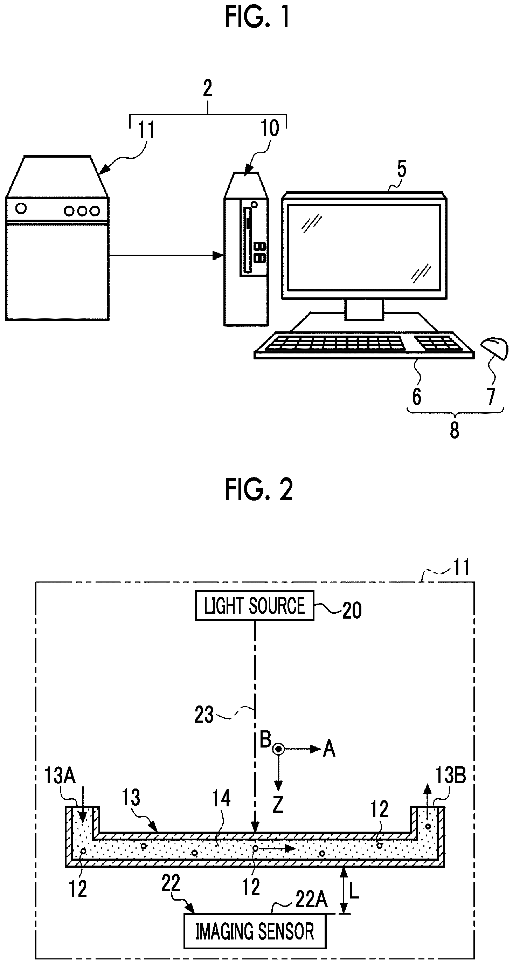

Fig. 1 shows a configuration of a digital holography system 2 which is an example of an imaging system. The digital holography system 2 is configured of an information processing device 10 and an imaging device 11. The imaging device 11 is connected to the information processing device 10.

The information processing device 10 is, for example, a desktop personal computer. A display 5, a keyboard 6, a mouse 7, and the like are connected to the information processing device 10. The keyboard 6 and the mouse 7 constitute an input device 8 for a user to input information. The input device 8 also includes a touch panel and the like.

Fig. 2 shows an example of a configuration of the imaging device 11. The imaging device 11 includes a light source 20 and an imaging sensor 22. The light source 20 is, for example, a laser diode. The light source 20 may be configured by combining a light emitting diode and a pinhole.

A microchannel 13 is disposed between the light source 20 and the imaging sensor 22. The microchannel 13 is formed in, for example, a channel unit formed of a silicone resin and is a flow channel through which a liquid can flow. The channel unit is transparent to light and can irradiate the inside of the microchannel 13 with light from the outside of the channel unit. The channel unit may be fixed in the imaging device 11 or may be attachable and detachable from the imaging device 11.

The microchannel 13 is provided with an opening portion 13A for introducing a solution 14 containing a cell 12 and the like and an opening portion 13B for discharging the solution 14 introduced into the microchannel 13. The microchannel 13 is an example of a "flow channel" according to the technique of the present disclosure.

The solution 14 is introduced into the opening portion 13A of the microchannel 13 from a tank (not shown), flows through the microchannel 13 at a constant speed, and is discharged from the opening portion 13B. The light source 20, the imaging sensor 22, and the microchannel 13 are disposed in, for example, an incubator (not shown). For example, the imaging device 11 performs imaging with the cell 12 contained in the solution 14 as an imaging target. The cell 12 is an example of an "object to be observed" according to the technique of the present disclosure.

The light source 20 irradiates irradiation light 23 toward the microchannel 13. The irradiation light 23 is coherent light. The irradiation light 23 is incident on the microchannel 13, passes through the microchannel 13, and then is incident on an imaging surface 22A of the imaging sensor 22. A Z direction indicated by an arrow is an irradiation direction of the irradiation light 23. In a case where the irradiation light 23 irradiated from the light source 20 is a luminous flux having a spread, the Z direction corresponds to a central axis direction of the luminous flux. The Z direction is an example of a "first direction" according to the technique of the present disclosure.

The microchannel 13 extends in an A direction orthogonal to the Z direction. The microchannel 13 is a flow channel through which the cell 12 as the object to be observed flows in the A direction. The A direction is an example of a "second direction" according to the technique of the present disclosure. In Fig. 2, a reference numeral B indicates a direction orthogonal to the Z direction and the A direction.

A shape of the microchannel 13 and the number of opening portions 13A and 13B can be changed as appropriate. Further, the number of microchannels 13 disposed between the light source 20 and the imaging sensor 22 is not limited to one and may be two or more. In the present embodiment, one microchannel 13 is assumed to be disposed between the light source 20 and the imaging sensor 22.

The imaging sensor 22 is configured of, for example, a monochrome complementary metal oxide semiconductor (CMOS) type image sensor. An imaging operation of the imaging sensor 22 is controlled by the information processing device 10. The imaging sensor 22 is disposed such that the imaging surface 22A is orthogonal to the Z direction. It is preferable that a distance L between the imaging surface 22A and the channel unit in which the microchannel 13 is formed is as small as possible. Further, it is also preferable that the imaging surface 22A is in contact with the channel unit (that is, L = 0).

The irradiation light 23 is incident on the solution 14 in the microchannel 13 and diffracted by the cell 12, and thus an interference fringe reflecting a shape of the cell 12 is generated.

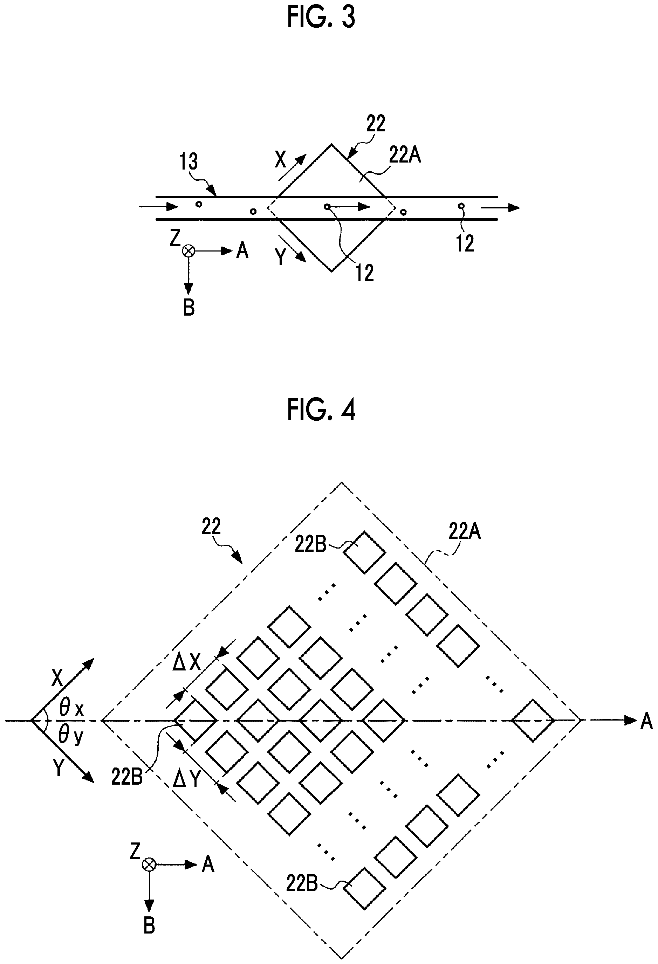

Fig. 3 shows an example of a positional relationship between the microchannel 13 and the imaging sensor 22 in a plan view. The imaging sensor 22 has a rectangular outer shape in a plan view. The imaging sensor 22 is disposed in an inclined state such that each side of the imaging sensor 22 is at an angle of 45° with respect to the A direction in which the cell 12 flows through the microchannel 13.

Fig. 4 shows an example of a pixel arrangement of the imaging sensor 22. The imaging sensor 22 has a plurality of pixels 22B arranged on the imaging surface 22A. The pixel 22B is a photoelectric conversion element that performs photoelectric conversion of the incident light to output a pixel signal according to an amount of incident light. The plurality of pixels 22B are two-dimensionally arranged on the imaging surface 22A in a manner non-parallel to the A direction.

The pixels 22B are arranged at equal pitches along an X direction and a Y direction. The arrangement of the pixels 22B is a so-called square arrangement. The X direction is a direction orthogonal to the Z direction. The Y direction is a direction orthogonal to the X direction and the Z direction. In the present embodiment, the X direction and the Y direction are respectively parallel to two orthogonal sides of the outer shape of the imaging sensor 22 in a plan view (refer to Fig. 3). That is, an angle $\theta x$ formed by the X direction with the A direction and an angle $\theta y$ formed by the Y direction with the A direction are each 45°.

The pixels 22B are arranged in the X direction at a first arrangement pitch $\Delta X$ and in the Y direction at a second arrangement pitch $\Delta Y$. In the present embodiment, the first arrangement pitch $\Delta X$ is equal to the second arrangement pitch $\Delta Y$. That is, in the present embodiment, the arrangement of the pixels 22B is the so-called square arrangement.

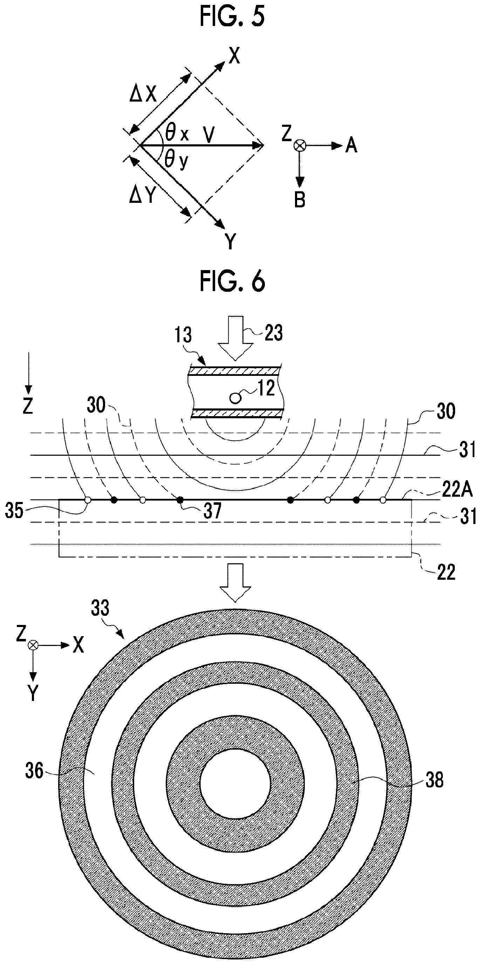

As shown in Fig. 5, a diagonal direction vector V with the first arrangement pitch $\Delta X$ as an X-direction component and the second arrangement pitch $\Delta Y$ as a Y-direction component is parallel to the A direction.

The imaging sensor 22 images the light incident on the imaging surface 22A and outputs image data configured of the pixel signal output from each of the pixels 22B. Hereinafter, the output of the image data is simply referred to as the output of the image.

Fig. 6 shows a state in which an interference fringe is generated by the cell 12 as the object to be observed. A part of the irradiation light 23 incident on the microchannel 13 is diffracted by the cell 12. That is, the irradiation light 23 is divided into diffracted light 30 diffracted by the cell 12 and transmitted light 31 that is not diffracted by the cell 12 and transmits through the microchannel 13. The transmitted light 31 is a planar wave. The diffracted light 30 and the transmitted light 31 pass through the microchannel 13 and are incident on the imaging surface 22A of the imaging sensor 22.

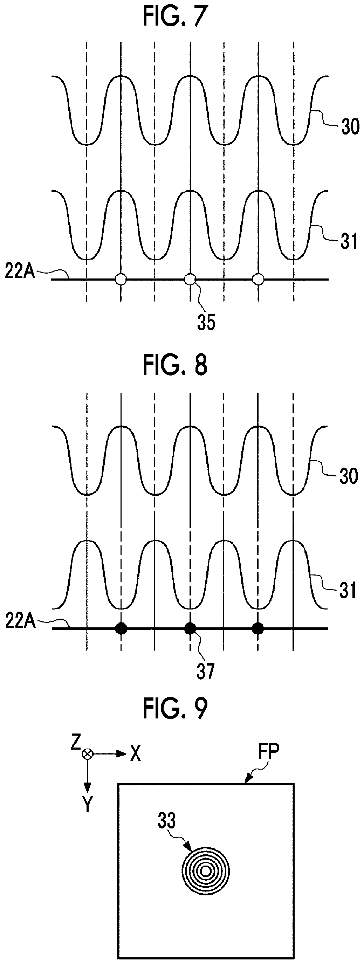

The diffracted light 30 and the transmitted light 31 interfere with each other to generate an interference fringe 33. The interference fringe 33 is configured of a bright portion 36 and a dark portion 38. In Fig. 6, the bright portion 36 and the dark portion 38 are illustrated in the interference fringe 33 as circular portions, respectively. However, the shape of the interference fringe 33 changes according to the shape and internal structure of the cell 12. The imaging sensor 22 captures an optical image including the interference fringes 33 formed on the imaging surface 22A and outputs an interference fringe image FP (refer to Fig. 7) including the interference fringes 33. The interference fringe image FP is also referred to as a hologram image.

Figs. 7 and 8 show wavefronts of the diffracted light 30 and the transmitted light 31. Fig. 7 shows the wavefront in a case where the diffracted light 30 and the transmitted light 31 strengthen each other. Fig. 8 shows the wavefront in a case where the diffracted light 30 and the transmitted light 31 weaken each other. In Figs. 7 and 8, solid lines indicate the wavefronts having a maximum amplitude of the diffracted light 30 and the transmitted light 31. On the contrary, broken lines indicate the wavefront having a minimum amplitude of the diffracted light 30 and the transmitted light 31.

In Fig. 7, a white spot 35 shown on the imaging surface 22A is a portion where the wavefronts of the diffracted light 30 and the transmitted light 31 are aligned and strengthen each other. The portion of the white spot 35 corresponds to the bright portion 36 (refer to Fig. 6) of the interference fringe 33. In Fig. 8, a black spot 37 shown on the imaging surface 22A is a portion where the wavefronts of the diffracted light 30 and the transmitted light 31 are deviated by a half wavelength and weaken each other. The portion of the black spot 37 corresponds to the dark portion 38 (refer to Fig. 6) of the interference fringe 33.

Fig. 9 shows an example of the interference fringe image FP output from the imaging sensor 22. The interference fringe image FP shown in Fig. 9 includes one interference fringe 33 generated by the diffraction of the irradiation light 23 by one cell 12 (refer to Fig. 6) included in an imaging region of the imaging sensor 22.

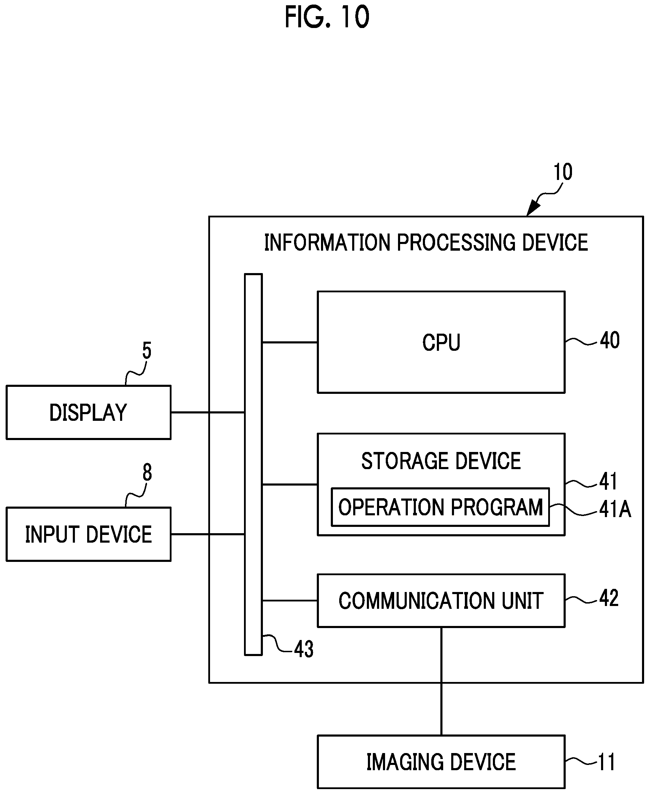

Fig. 10 shows an example of a hardware configuration of the information processing device 10. As shown in Fig. 10, the information processing device 10 comprises a central processing unit (CPU) 40, a storage device 41, and a communication unit 42, which are interconnected via a bus line 43. Further, the display 5 and the input device 8 are connected to the bus line 43.

The CPU 40 is a calculation device that reads out an operation program 41A and various types of data (not shown) stored in the storage device 41 and executes processing to realize various functions. The CPU 40 is an example of a "processor" according to the technique of the present disclosure.

The storage device 41 includes, for example, a random access memory (RAM), a read only memory (ROM), or a storage device. The RAM is, for example, a volatile memory used as a work area or the like. The ROM is, for example, a non-volatile memory such as a flash memory that holds the operation program 41A and various types of data. The storage device is, for example, a hard disk drive (HDD) or a solid state drive (SSD). The storage stores an operating system (OS), an application program, image data, various types of data, and the like.

The communication unit 42 is a network interface that controls transmission of various types of information via a network such as a local area network (LAN) or a wide area network (WAN). The information processing device 10 is connected to the imaging device 11 via the communication unit 42. The display 5 displays various screens. The information processing device 10 receives an input of an operation instruction from the input device 8 through various screens.

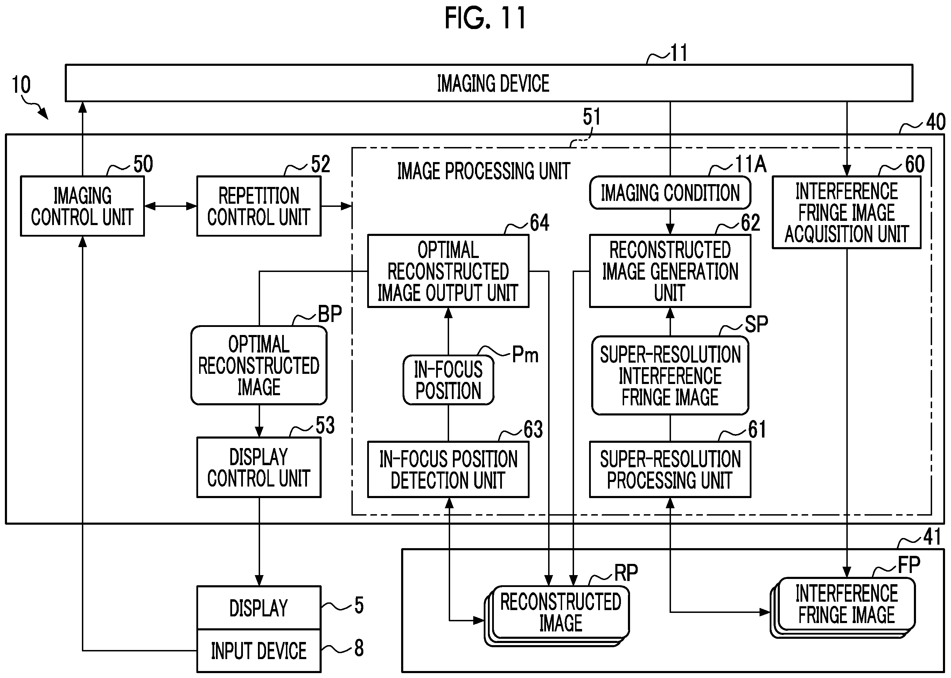

Fig. 11 shows an example of a functional configuration of the information processing device 10. A function of the information processing device 10 is realized by the CPU 40 executing processing based on the operation program 41A. As shown in Fig. 11, the CPU 40 is configured of an imaging control unit 50, an image processing unit 51, a repetition control unit 52, and a display control unit 53.

The imaging control unit 50 controls an operation of the imaging device 11. Specifically, the imaging control unit 50 controls an operation of generating the irradiation light 23 by the light source 20 and an imaging operation of the imaging sensor 22. Hereinafter, the operation of generating the irradiation light 23 by the light source 20 and the imaging operation of the imaging sensor 22 are collectively referred to as an imaging operation of the imaging device 11. The imaging control unit 50 causes the imaging device 11 to execute the imaging operation based on an operation signal input from the input device 8.

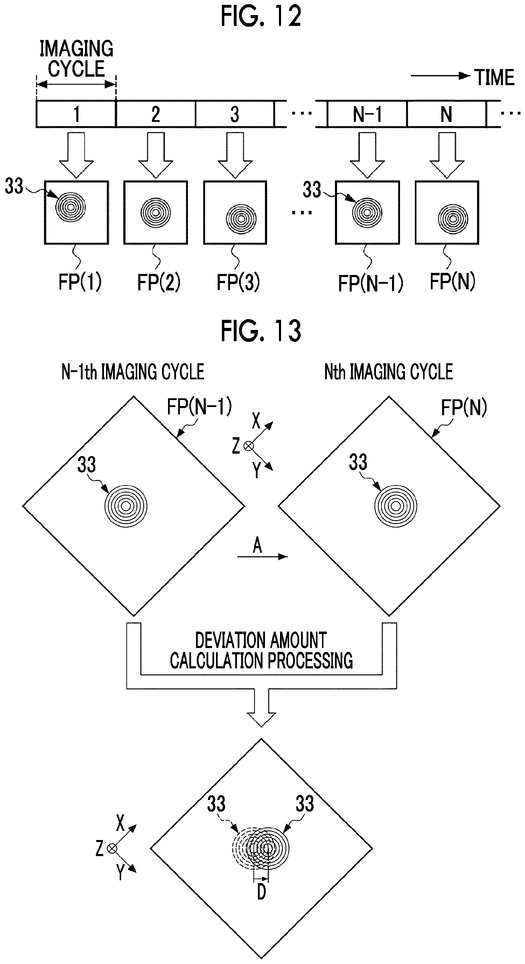

The imaging control unit 50 drives the imaging device 11 to periodically perform the imaging every one imaging cycle. That is, the imaging device 11 captures the moving image. As shown in Fig. 12, the imaging device 11 performs the imaging operation every one imaging cycle and outputs the interference fringe image FP. An interference fringe image FP(N) represents an interference fringe image FP output from the imaging device 11 in an Nth imaging cycle.

Here, N is a positive integer. Hereinafter, in a case where it is not necessary to distinguish between the imaging cycles, the interference fringe image is simply referred to as the interference fringe image FP.

The image processing unit 51 performs reconstruction processing, in-focus position detection processing, and the like based on the interference fringe image FP (refer to Fig. 9) output from the imaging device 11, and outputs an optimal reconstructed image BP in which the cell 12, which is the object to be observed, is in focus.

The repetition control unit 52 causes the image processing unit 51 to repeatedly execute the reconstruction processing, the in-focus position detection processing, and the like in synchronization with the imaging cycle of the imaging device 11. The image processing unit 51 outputs the optimal reconstructed image BP every one imaging cycle.

The display control unit 53 displays the optimal reconstructed image BP output from the image processing unit 51 every one imaging cycle on the display 5. Accordingly, the optimal reconstructed image BP is displayed on the display 5 in real time.

The imaging control unit 50 causes the imaging device 11 to start the imaging operation in response to an input of an imaging start signal from the input device 8 and to stop the imaging operation of the imaging device 11 in response to an input of an imaging stop signal from the input device 8. The repetition control unit 52 causes the image processing unit 51 to start the operation in response to the start of the imaging operation by the imaging device 11 and to stop the operation of the image processing unit 51 in response to the stop of the imaging operation.

The image processing unit 51 includes an interference fringe image acquisition unit 60, a super-resolution processing unit 61, a reconstructed image generation unit 62, an in-focus position detection unit 63, and an optimal reconstructed image output unit 64.

The interference fringe image acquisition unit 60 acquires the interference fringe image FP (refer to Fig. 12) output as a result of imaging of the microchannel 13 by the imaging device 11 every one imaging cycle. The interference fringe image acquisition unit 60 stores the acquired interference fringe image FP in the storage device 41.

The super-resolution processing unit 61 generates a super-resolution interference fringe image SP based on the plurality of interference fringe images FP stored in the storage device 41. Specifically, the super-resolution processing unit 61 generates the super-resolution interference fringe image SP based on two interference fringe images FP output from the imaging sensor 22 in two consecutive imaging cycles and a deviation amount of the interference fringe 33 included in each interference fringe image FP.

Fig. 13 schematically shows deviation amount calculation processing. As shown in Fig. 13, the super-resolution processing unit 61 acquires, from the storage device 41, the interference fringe image FP(N) and an interference fringe image FP(N-1) output from the imaging sensor 22 in immediately preceding two imaging cycles. The interference fringe image FP(N) is an interference fringe image FP output from the imaging sensor 22 in the Nth imaging cycle. The interference fringe image FP(N-1) is an interference fringe image FP output from the imaging sensor 22 in an N-1th imaging cycle.

The super-resolution processing unit 61 performs image matching between the interference fringe image FP(N) and the interference fringe image FP(N-1) using a method based on image analysis such as a phase-limited correlation method to calculate a deviation amount D of the interference fringe 33. The deviation amount D corresponds to an amount of movement of the cell 12 in the A direction in one imaging cycle.

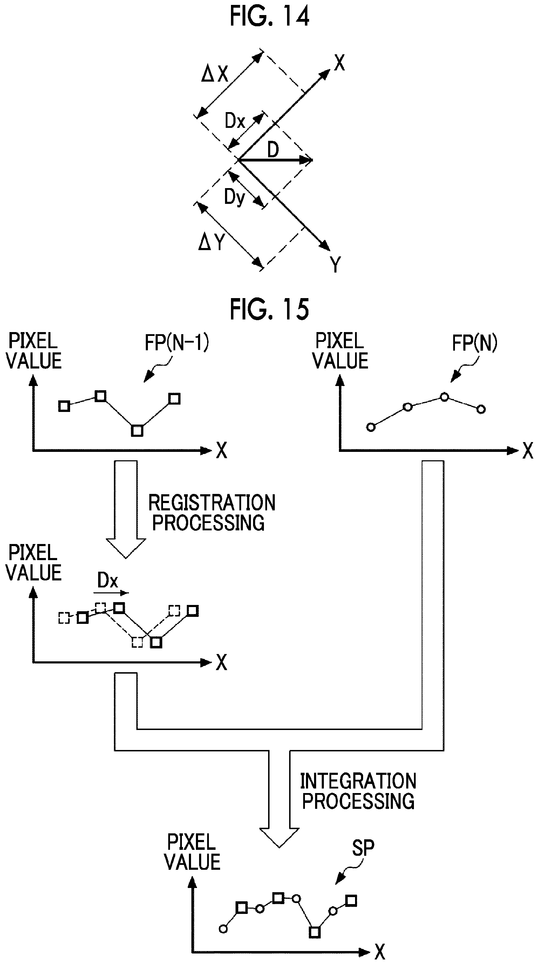

In order to perform super-resolution processing, as shown in Fig. 14, as for the deviation amount D, a component Dx in the X direction needs to be a non-integer multiple of the first arrangement pitch $\Delta X$, and a component Dy in the Y direction needs to be a non-integer multiple of the second arrangement pitch $\Delta Y$. Hereinafter, the component Dx in the X direction is referred to as an "X-direction component Dx", and the component Dy in the Y direction is referred to as a "Y-direction component Dy". Fig. 14 shows a case where the X-direction component Dx is smaller than the first arrangement pitch $\Delta X$ and the Y-direction component Dy is smaller than the second arrangement pitch $\Delta Y$. The X-direction component Dx and the Y-direction component Dy may be larger than the first arrangement pitch $\Delta X$ and the second arrangement pitch $\Delta Y$ as long as the X-direction component Dx and the Y-direction component Dy are respectively the non-integer multiples of the first arrangement pitch $\Delta X$ and the second arrangement pitch $\Delta Y$.

Although it is difficult to control a speed of the cell 12 flowing through the microchannel 13 to precisely control the deviation amount D, it is possible to easily obtain the deviation amount D with the image analysis of the two interference fringe images FP using the method such as the phase-limited correlation method as described above.

The interference fringe image FP(N) and the interference fringe image FP(N-1) correspond to two images in so-called "pixel shift" according to the super-resolution technique, and the deviation amount D corresponds to a pixel shift amount. The pixel shift technique is known in JP1975-17134 (JP-S50-17134), JP2001-111879, and the like.

The super-resolution processing unit 61 obtains the deviation amount D, then registers the interference fringe image FP(N) and the interference fringe image FP(N-1) based on the deviation amount D, and integrates the interference fringe image FP(N) and the interference fringe image FP(N-1) after the registration to generate the super-resolution interference fringe image SP.

Fig. 15 schematically shows registration processing and integration processing. Fig. 15 illustrates changes in pixel values of the interference fringe image FP(N) and the interference fringe image FP(N-1) in the X direction. The super-resolution processing unit 61 moves, for example, the interference fringe image FP(N-1) among the interference fringe image FP(N) and the interference fringe image FP(N-1) based on the deviation amount D to perform the registration. Specifically, the super-resolution processing unit 61 moves the interference fringe image FP(N-1) by the X-direction component Dx of the deviation amount D in the X direction, and moves the interference fringe image FP(N-1) by the X-direction component Dx of the deviation amount D in the Y direction. Although Fig. 15 shows only the registration in the X direction, the registration in the Y direction is also performed in the same manner.

The super-resolution processing unit 61 performs the integration processing of integrating the interference fringe image FP(N) and the interference fringe image FP(N-1) which are subjected to the registration. Accordingly, the super-resolution interference fringe image SP whose resolution is doubled with respect to the interference fringe image FP is generated. In a simple integration processing, pixels of the super-resolution interference fringe image SP may not be arranged at equal intervals. Thus, processing of equalizing intervals of the pixel arrangements of the super-resolution interference fringe images SP may be added.

As described above, since the plurality of pixels 22B are two-dimensionally arranged on the imaging surface 22A in a manner non-parallel to the A direction, there are finite non-zero components of the X-direction component Dx and the Y-direction component Dy in the deviation amount D. Therefore, the resolution of the super-resolution interference fringe image SP increases in the X direction and the Y direction with respect to the interference fringe image FP. In a case where the X-direction component Dx is equal to the Y-direction component Dy, a resolution imbalance that occurs in the X direction and the Y direction is reduced and equalized in the super-resolution interference fringe image SP. Therefore, it is preferable that the angles $\theta x$ and $\theta y$ (refer to Fig. 4) are each set to 45°.

The super-resolution processing unit 61 is not limited to the two interference fringe images FP, and may use three or more interference fringe images FP to generate the super-resolution interference fringe image SP. The resolution of the super-resolution interference fringe image SP to be generated increases as more interference fringe images FP are used, while a processing load increases. Therefore, it is preferable to decide the number of interference fringe images FP used in the super-resolution processing according to an allowable processing load.

Returning to Fig. 11, the reconstructed image generation unit 62 reconstructs the super-resolution interference fringe image SP generated by the super-resolution processing unit 61 to generate the reconstructed image RP and stores the generated reconstructed image RP in the storage device 41. The super-resolution interference fringe image SP is input to the reconstructed image generation unit 62 from the super-resolution processing unit 61 every one imaging cycle. The reconstructed image generation unit 62 generates the plurality of reconstructed images RP for one input super-resolution interference fringe image SP while changing the reconstruction position.

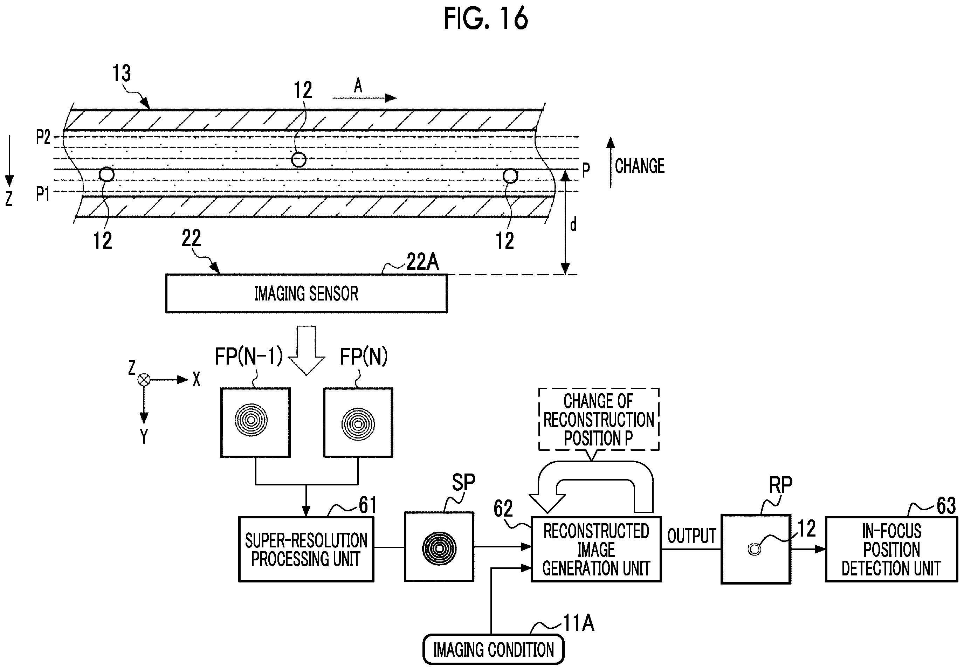

Specifically, as shown in Fig. 16, the reconstructed image generation unit 62 generates the reconstructed image RP each time the reconstruction position P is changed while changing the reconstruction position P by a constant value. The reconstruction position P is a position (so-called depth position) represented by a distance d from the imaging surface 22A of the imaging sensor 22 in a direction of the light source 20.

The reconstructed image generation unit 62 performs the reconstruction processing based on, for example, Fresnel conversion equations represented by the following equations (1) to (3).

$$\Gamma(m,n) = \frac{i}{\lambda d} \exp\left(-i\frac{2\pi}{\lambda}d\right) \exp\left[-i\pi\lambda d\left(\frac{m^2}{N_x^2 \Delta x^2} + \frac{n^2}{N_y^2 \Delta y^2}\right)\right]$$

$$\times \sum_{x=0}^{N_x-1}\sum_{y=0}^{N_y-1} I(x,y)\exp\left[-i\frac{\pi}{\lambda d}(x^2\Delta x^2 + y^2\Delta y^2)\right]\exp\left[i2\pi\left(\frac{xm}{N_x^2} + \frac{yn}{N_y^2}\right)\right] \cdots (1)$$

$$A_0(m,n) = |\Gamma(m,n)|^2 \cdots (2)$$

$$\varphi_0(m,n) = \arctan\frac{\mathrm{Im}[\Gamma(m,n)]}{\mathrm{Re}[\Gamma(m,n)]} \cdots (3)$$

Here, I(x,y) represents the super-resolution interference fringe image SP. x represents an X coordinate of the pixel of the super-resolution interference fringe image SP. y represents a Y coordinate of the pixel of the super-resolution interference fringe image SP. $\Delta x$ is an arrangement pitch of the pixels of the super-resolution interference fringe image SP in the X direction. $\Delta y$ is an arrangement pitch of the pixels of the super-resolution interference fringe image SP in the Y direction. A is a wavelength of the irradiation light 23.

As shown in equation (1), $\Gamma(m,n)$ is a complex amplitude image obtained by performing the Fresnel conversion on the super-resolution interference fringe image SP. Here, m = 1, 2, 3, ..., and Nx-1, and n = 1, 2, 3, ... , and Ny-1. Nx represents the number of pixels of the super-resolution interference fringe image SP in the X direction. Ny represents the number of pixels of the super-resolution interference fringe image SP in the Y direction.

As shown in equation (2), $A_0(m,n)$ is an intensity distribution image representing an intensity component of the complex amplitude image $\Gamma(m,n)$. As shown in equation (3), $\varphi_0(m,n)$ is a phase distribution image representing a phase component of the complex amplitude image $\Gamma(m,n)$.

The reconstructed image generation unit 62 obtains the complex amplitude image $\Gamma(m,n)$ by applying the super-resolution interference fringe image SP to equation (1) and obtains the intensity distribution image $A_0(m,n)$ or the phase distribution image $\varphi_0(m,n)$ by applying the obtained complex amplitude image $\Gamma(m,n)$ to equation (2) or equation (3). The reconstructed image generation unit 62 obtains any one of the intensity distribution image $A_0(m,n)$ or the phase distribution image $\varphi_0(m,n)$, outputs the obtained image as the reconstructed image RP and stores the obtained image in the storage device 41.

In the present embodiment, the reconstructed image generation unit 62 outputs the phase distribution image $\varphi_0(m,n)$ as the reconstructed image RP. The phase distribution image $\varphi_0(m,n)$ is an image representing a refractive index distribution of the object to be observed. The cell 12 which is the object to be observed in the present embodiment is translucent, and thus most of the irradiation light 23 is not absorbed by the cell 12, but is transmitted or diffracted. Therefore, an image hardly appears in an intensity distribution. Therefore, in the present embodiment, it is preferable to use the phase distribution image $\varphi_0(m,n)$ as the reconstructed image RP.

The wavelength $\lambda$ of the irradiation light 23 is included in, for example, an imaging condition 11A supplied from the imaging device 11. The reconstructed image generation unit 62 performs the calculation of equation (1) using a value of the wavelength $\lambda$ included in the imaging condition 11A. Further, the reconstructed image generation unit 62 obtains the complex amplitude image $\Gamma(m,n)$ by performing the calculation of equation (1) while changing the distance d corresponding to the reconstruction position P by a constant value, and applies the obtained complex amplitude image $\Gamma(m,n)$ to equation (2) or equation (3).

The reconstructed image generation unit 62 changes the reconstruction position P by a constant value within a range from a lower limit position P1 to an upper limit position P2. The reconstructed image generation unit 62 starts the change of the reconstruction position P, for example, with the lower limit position P1 as an initial position. The change of the reconstruction position P corresponds to the change of the distance d in equation (1).

In the reconstructed image generation unit 62, the reconstruction processing method is not limited to the method using the Fresnel conversion equation and the reconstruction processing may be performed by a Fourier iterative phase recovery method or the like.

Returning to Fig. 11, the in-focus position detection unit 63 obtains the sharpness of each reconstructed image RP that is output from the reconstructed image generation unit 62 and stored in the storage device 41 to search for the reconstruction position P (hereinafter in-focus position Pm) where the sharpness is maximized. The in-focus position detection unit 63 detects the in-focus position Pm and inputs the in-focus position Pm to the optimal reconstructed image output unit 64 every one imaging cycle.

The in-focus position detection unit 63 calculates, for example, a contrast value of the reconstructed image RP as the sharpness. The in-focus position detection unit 63 may use a value obtained by evaluating the spread of the image of the cell 12 in the reconstructed image RP with a cross-sectional profile or the like as the sharpness. Further, the in-focus position detection unit 63 may perform frequency analysis such as Fourier analysis to obtain the sharpness.

The optimal reconstructed image output unit 64 acquires the reconstructed image RP corresponding to the in-focus position Pm from the storage device 41 each time the in-focus position Pm is detected by the in-focus position detection unit 63 every one imaging cycle. Further, the optimal reconstructed image output unit 64 performs optimal reconstructed image output processing of outputting the acquired reconstructed image RP to the display control unit 53 as the optimal reconstructed image BP.

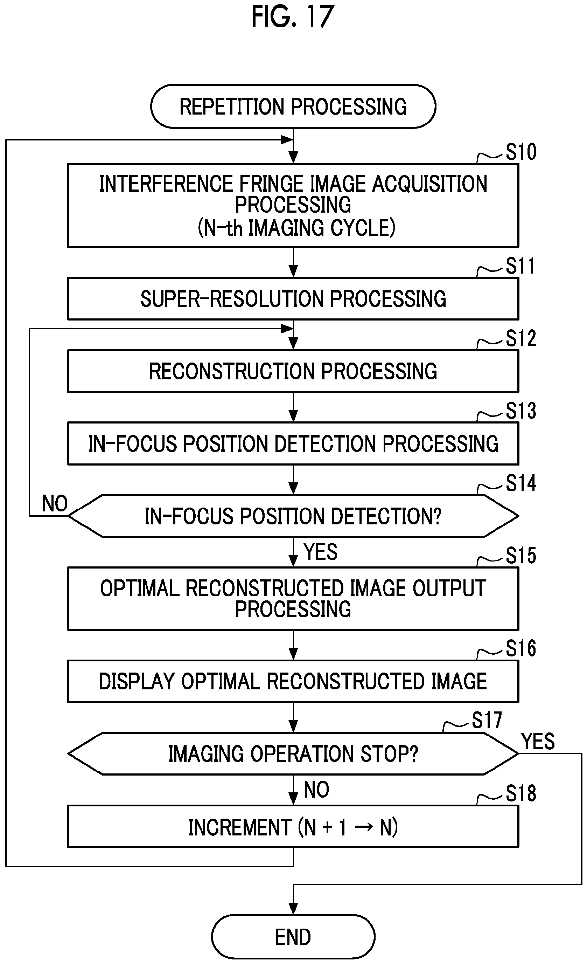

Fig. 17 shows an example of a flow of repetition processing by the repetition control unit 52. In a case where the imaging operation (refer to Fig. 12) by the imaging device 11 is started, the interference fringe image acquisition unit 60 acquires the interference fringe image FP(N) corresponding to the Nth imaging cycle (step S10). The interference fringe image FP(N) acquired by the interference fringe image acquisition unit 60 is stored in the storage device 41. Here, the storage device 41 is assumed to already store the interference fringe image FP(N-1) corresponding to the N-1th imaging cycle.

The super-resolution processing unit 61 reads the interference fringe image FP(N) and the interference fringe image FP(N-1) from the storage device 41 and performs the deviation amount calculation processing (refer to Fig. 13), the registration processing, and the integration processing (refer to Fig. 15) to generate the super-resolution interference fringe image SP (step S11).

Next, the reconstructed image generation unit 62 sets the reconstruction position P to the initial position based on the super-resolution interference fringe image SP generated by the super-resolution processing unit 61 and then performs the above reconstruction processing to generate the reconstructed image RP (step S12). In step S12, the reconstructed image RP for one reconstruction position P is generated and stored in the storage device 41.

Next, the in-focus position detection unit 63 reads the reconstructed image RP from the storage device 41, calculates the sharpness of the reconstructed image RP, and detects the in-focus position Pm based on the calculated sharpness (step S13). Since the in-focus position Pm is the reconstruction position P where the sharpness is maximized, it is necessary to calculate the sharpness for at least three reconstructed images RP for the detection of the in-focus position Pm. For this purpose, step S13 needs to be repeated at least three times.

The repetition control unit 52 determines whether or not the in-focus position Pm is detected by the in-focus position detection unit 63 (step S14). In a case where the in-focus position Pm is determined to be not detected (step S14: NO), the repetition control unit 52 returns the processing to step S12. In step S12, the reconstructed image generation unit 62 changes the reconstruction position P by a certain value and then the reconstructed image RP is generated again. Each of the pieces of processing of step S12 and step S13 is repeatedly executed until the determination is affirmed in step S14.

In a case where the in-focus position Pm is detected by the in-focus position detection unit 63 (step S14: YES), the repetition control unit 52 shifts the processing to step S15. In step S15, the optimal reconstructed image output unit 64 acquires the reconstructed image RP corresponding to the in-focus position Pm detected by the in-focus position detection unit 63 from the storage device 41 and outputs the acquired reconstructed image RP as the optimal reconstructed image BP to the display control unit 53 (step S15).

The display control unit 53 displays the optimal reconstructed image BP input from the optimal reconstructed image output unit 64 on the display 5 (step S16).

Next, the repetition control unit 52 determines whether or not the imaging stop signal is input from the input device 8 (step S17). In a case where the imaging stop signal is determined to be not input (step S17: NO), the repetition control unit 52 increments the parameter N (refer to Fig. 12) representing the imaging cycle number (step S18) and returns the processing to step S10. In step S10, the interference fringe image acquisition unit 60 acquires an interference fringe image FP(N+1) corresponding to an N+1th imaging cycle. In step S11, the super-resolution processing unit 61 generates the super-resolution interference fringe image SP based on the interference fringe image FP(N+1) and the interference fringe image FP(N). Each of the pieces of processing from step S10 to step S18 is repeatedly executed every one imaging cycle until the determination is affirmed in step S17.

In a case where the imaging stop signal is determined to be input from the input device 8 (step S17: YES), the repetition control unit 52 ends the series of pieces of repetition processing.

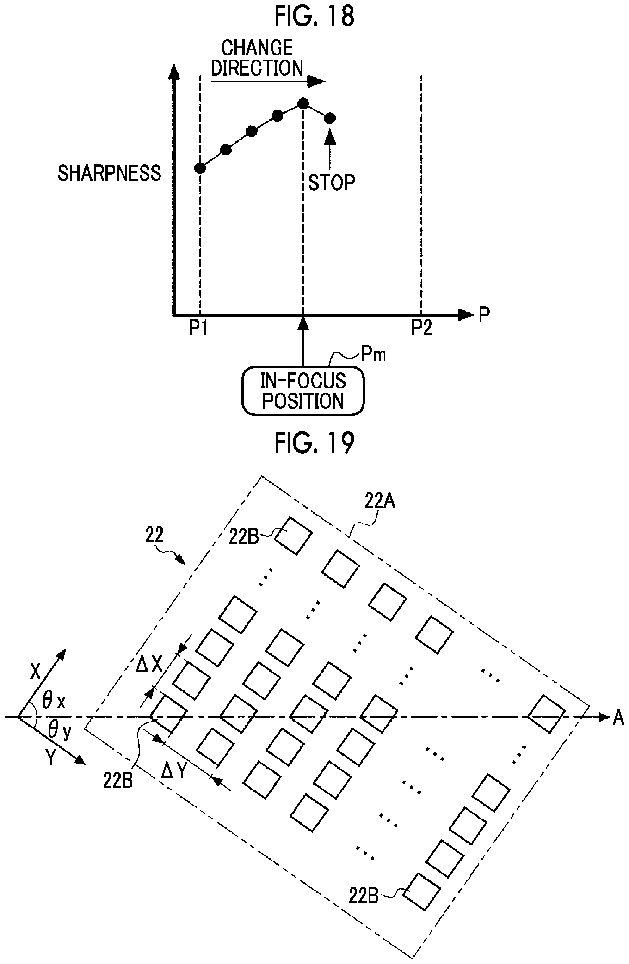

Fig. 18 shows an example of processing of searching for the in-focus position Pm executed by the in-focus position detection unit 63 in step S13 of Fig. 17. As shown in Fig. 18, the in-focus position detection unit 63 performs, for example, peak determination of the sharpness by a so-called mountain climbing method. Each time the sharpness is calculated, the in-focus position detection unit 63 plots the calculated sharpness in association with the reconstruction position P. The sharpness increases as the reconstruction position P approaches an in-focus position Pm and decreases after the reconstruction position P passes the in-focus position Pm. In a case where detection is made that the sharpness has changed from the increase to the decrease, the in-focus position detection unit 63 detects a previous reconstruction position P as the in-focus position Pm. The in-focus position Pm corresponds to a depth position of the cell 12, which is the object to be observed.

As described above, according to the technique of the present disclosure, with the two-dimensional arrangement of the plurality of pixels 22B in a manner non-parallel to the A direction in which the cell 12 which is the object to be observed flows, the plurality of interference fringe images FP in which positions of the interference fringes 33 caused by the cell 12 are deviated in the X direction and the Y direction are obtained. The plurality of interference fringe images FP are subjected to the super-resolution processing to obtain the super-resolution interference fringe image SP. Therefore, according to the technique of the present disclosure, it is possible to generate the super-resolution interference fringe image of the object to be observed flowing through the flow channel.

Further, according to the technique of the present disclosure, with the reconstruction of the super-resolution interference fringe image SP and the detection of the reconstructed image RP at the reconstruction position P where the sharpness is maximized, it is possible to acquire the optimal reconstructed image BP with high definition.

[Modification Example]

Hereinafter, various modification examples will be described. In the above embodiment, as shown in Fig. 4, the imaging sensor 22 in which the first arrangement pitch $\Delta X$ is equal to the second arrangement pitch $\Delta Y$ is used. However, the imaging sensor 22 in which the first arrangement pitch $\Delta X$ is different from the second arrangement pitch $\Delta Y$ may be used.

Fig. 19 shows a modification example of the imaging sensor 22. Fig. 19 shows an imaging sensor 22 in which the second arrangement pitch $\Delta Y$ is longer than the first arrangement pitch $\Delta X$. In this case, the angle $\theta x$ formed by the X direction with the A direction and the angle $\theta y$ formed by the Y direction with the A direction are set to angles other than 45°.

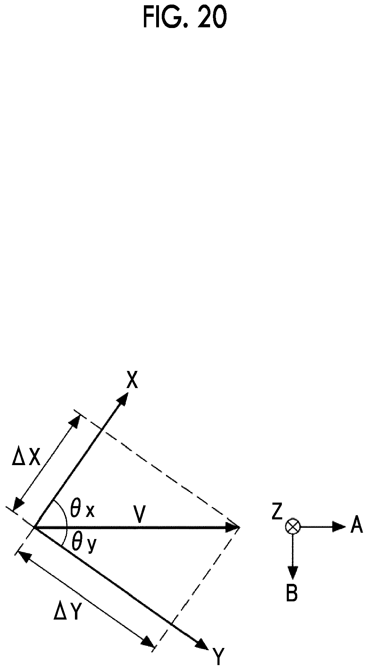

Specifically, as shown in Fig. 20, the angles $\theta x$ and $\theta y$ may be decided such that the diagonal direction vector V with the first arrangement pitch $\Delta X$ as the X-direction component and the second arrangement pitch $\Delta Y$ as the Y-direction component is parallel to the A direction.

That is, the angles $\theta x$ and $\theta y$ that satisfy the following equations (4) and (5) may be obtained. Further, in the following equations (4) and (5), the angle is represented by a radian.

$$\theta_x = \arctan\frac{\Delta Y}{\Delta X} \quad \cdots (4)$$

$$\theta_y = \frac{\pi}{2} - \arctan\frac{\Delta Y}{\Delta X} \quad \cdots (5)$$

Accordingly, even in a case where the first arrangement pitch $\Delta X$ is different from the second arrangement pitch $\Delta Y$, the resolution imbalance that occurs in the X direction and the Y direction is reduced and equalized in the super-resolution interference fringe image SP.

In the above embodiment, the phase distribution image $\varphi_0(m,n)$ obtained by equation (3) is used as the reconstructed image RP, but the reconstructed image RP is not limited thereto. The intensity distribution image $A_0(m,n)$ obtained by equation (2) may be used as the reconstructed image RP. In a case where the object to be observed has a thickness such as a cell population (so-called colony), an image appears in the intensity distribution. Therefore, it is preferable to use the intensity distribution image $A_0(m,n)$ as the reconstructed image RP.

The user may select which of the phase distribution image $\varphi_0(m,n)$ and the intensity distribution image $A_0(m,n)$ is used as the reconstructed image RP, by using the input device 8. Accordingly, the user can select an optimal reconstructed image RP according to the object to be observed.

In the above embodiment, although the object to be observed is the cell, the object to be observed is not limited to the cell and may be a dead cell or an object such as dust.

The digital holography system 2 according to the above embodiment relates to a technique referred to as so-called lens-free imaging in which the imaging device 11 does not comprise an optical lens. The technique of the present disclosure is not limited to the lens-free imaging and can be applied to general digital holography (for example, in a case where reference light is used).

The hardware configuration of the computer configuring the information processing device 10 may be modified in various ways. For example, the information processing device 10 may be configured of a plurality of computers separated as hardware for the purpose of improving processing capacity and reliability.

As described above, the hardware configuration of the computer of the information processing device 10 may be changed as appropriate according to required performance such as processing capacity, safety, and reliability. Further, not only the hardware but also the application program such as the operation programs 41A may be duplicated or stored in a plurality of storage devices in a distributed manner for the purpose of ensuring safety and reliability.

In the above embodiment, for example, as a hardware structure of the processing units executing various types of processing such as the imaging control unit 50, the image processing unit 51, the repetition control unit 52, and the display control unit 53, various processors shown below can be used. The various processors include a programmable logic device (PLD) which is a processor whose circuit configuration is changeable after manufacturing such as a field programmable gate array (FPGA), a dedicated electric circuit which is a processor having a circuit configuration exclusively designed to execute specific processing such as an application specific integrated circuit (ASIC), and the like, in addition to the CPU 40 which is a general-purpose processor that executes software (operation program 41A) to function as various processing units, as described above.

One processing unit may be configured by one of the various processors or a combination of two or more processors of the same type or different types (for example, a combination of a plurality of FPGAs and/or a combination of a CPU and an FPGA). The plurality of processing units may be configured of one processor.

As an example of configuring the plurality of processing units with one processor, first, there is a form in which one processor is configured by a combination of one or more CPUs and software and the processor functions as the plurality of processing units, as represented by computers such as a client and a server. Second, there is a form in which a processor that realizes the functions of the entire system including the plurality of processing units with one integrated circuit (IC) chip is used, as represented by a system on chip (SoC) or the like. As described above, the various processing units are configured using one or more of the various processors as the hardware structure.

Further, more specifically, a circuitry combining circuit elements such as semiconductor elements can be used as the hardware structure of the various processors.

The above embodiment and each modification example can be combined as appropriate as long as there is no contradiction.

In a case where all of documents, patent applications, and technical standard described in the specification are incorporated in the specification as references, to the same degree as a case where the incorporation of each of documents, patent applications, and technical standard as references is specifically and individually noted.

Explanation of References

2: digital holography system
5: display
6: keyboard
7: mouse
8: input device
10: information processing device
11: imaging device
11A: imaging condition
12: cell
13: microchannel
13A, 13B: opening portion
14: solution
20: light source
22: imaging sensor
22A: imaging surface
22B: pixel
23: irradiation light
30: diffracted light
31: transmitted light
33: interference fringe
35: white spot
36: bright portion
37: black spot
38: dark portion
40: CPU
41: storage device
41A: operation program
42: communication unit
43: bus line
50: imaging control unit
51: image processing unit
52: repetition control unit
53: display control unit
60: interference fringe image acquisition unit
61: super-resolution processing unit
62: reconstructed image generation unit
63: in-focus position detection unit
64: optimal reconstructed image output unit
$\Delta X$: arrangement pitch
$\Delta Y$: arrangement pitch
$\theta x$, $\theta y$: angle
BP: optimal reconstructed image
D: deviation amount
Dx: X direction component
Dy: Y direction component

FP: interference fringe image
L: distance
P1: lower limit position
P2: upper limit position
P: reconstruction position
Pm: in-focus position
RP: reconstructed image
SP: super-resolution interference fringe image
V: diagonal direction vector
d: distance

**Claims**

1.  An imaging system comprising:

    a light source that irradiates light in a first direction and irradiates light toward a flow channel through which an object to be observed flows in a second direction orthogonal to the first direction;
    an imaging sensor that has an imaging surface orthogonal to the first direction and on which a plurality of pixels are two-dimensionally arranged in a manner non-parallel to the second direction and that images light passing through the flow channel to output an interference fringe image; and
    an information processing device that generates a super-resolution interference fringe image based on a plurality of interference fringe images output from the imaging sensor.

2.  The imaging system according to claim 1, wherein in a case where one direction of an arrangement direction of the two-dimensional arrangement is an X direction and the other direction is a Y direction in the imaging surface, the plurality of pixels are arranged in the X direction at a first arrangement pitch and arranged in the Y direction orthogonal to the X direction at a second arrangement pitch.

3.  The imaging system according to claim 2, wherein a diagonal direction vector composed of the first arrangement pitch and the second arrangement pitch is parallel to the second direction.

4.  The imaging system according to claim 3, wherein the first arrangement pitch is equal to the second arrangement pitch.

5.  The imaging system according to any one of claims 2 to 4, wherein as for a deviation amount of two interference fringe images output from the imaging sensor in two consecutive imaging cycles, a component in the X direction is a non-integer multiple of the first arrangement pitch and a component in the Y direction is a non-integer multiple of the second arrangement pitch.

6.  The imaging system according to claim 5, wherein as for the deviation amount, the component in the X direction is smaller than the first arrangement pitch and the component in the Y direction is smaller than the second arrangement pitch.

7.  The imaging system according to claim 5 or 6, wherein the information processing device calculates the deviation amount based on the two interference fringe images output from the imaging sensor in the two consecutive imaging cycles and generates the super-resolution interference fringe image based on the calculated deviation amount and the two interference fringe images.

8.  The imaging system according to any one of claims 1 to 7, wherein the information processing device reconstructs the super-resolution interference fringe image to generate a reconstructed image.

9.  The imaging system according to claim 8, wherein the information processing device executes:

    reconstruction processing of generating the reconstructed image while changing a reconstruction position;
    in-focus position detection processing of calculating sharpness of the reconstructed image each time the reconstructed image is generated by the reconstruction processing and detecting an in-focus position where the calculated sharpness is maximized; and

optimal reconstructed image output processing of outputting the reconstructed image at the in-focus position detected by the in-focus position detection processing as an optimal reconstructed image.

10. An imaging device comprising:

a light source that irradiates light in a first direction and irradiates light toward a flow channel through which an object to be observed flows in a second direction orthogonal to the first direction; and
an imaging sensor that has an imaging surface orthogonal to the first direction and on which a plurality of pixels are two-dimensionally arranged in a manner non-parallel to the second direction and that images light passing through the flow channel to output an interference fringe image.

# FIG. 1

# FIG. 2

# FIG. 3

# FIG. 4

# FIG. 5

# FIG. 6

## FIG. 7

30

31

22A

35

## FIG. 8

30

31

22A

37

## FIG. 9

Z ⊗ → X

Y

FP

33

# FIG. 10

# FIG. 11

IMAGING DEVICE ~11

10

IMAGE PROCESSING UNIT ~51

IMAGING CONTROL UNIT ~50

REPETITION CONTROL UNIT ~52

IMAGING CONDITION ~11A

INTERFERENCE FRINGE IMAGE ACQUISITION UNIT ~60

~40

OPTIMAL RECONSTRUCTED IMAGE OUTPUT UNIT ~64

RECONSTRUCTED IMAGE GENERATION UNIT ~62

OPTIMAL RECONSTRUCTED IMAGE ~BP

IN-FOCUS POSITION ~Pm

SUPER-RESOLUTION INTERFERENCE FRINGE IMAGE ~SP

DISPLAY CONTROL UNIT ~53

IN-FOCUS POSITION DETECTION UNIT ~63

SUPER-RESOLUTION PROCESSING UNIT ~61

DISPLAY ~5

INPUT DEVICE ~8

RECONSTRUCTED IMAGE ~RP

INTERFERENCE FRINGE IMAGE ~FP

~41

EP 4 174 165 A1

# FIG. 12

IMAGING
CYCLE
→ TIME

| 1 | 2 | 3 | ⋯ | N−1 | N | ⋯ |

33

FP(1) FP(2) FP(3) ⋯ FP(N−1) FP(N)

33

# FIG. 13

N−1th IMAGING CYCLE          Nth IMAGING CYCLE

FP(N−1)    X          FP(N)

Z          Y

33                          33

A

DEVIATION AMOUNT
CALCULATION PROCESSING

X

Z          Y

33          33

D

# FIG. 14

# FIG. 15

FIG. 16

# FIG. 17

```
            ┌────────────────────────────────┐
            │     REPETITION PROCESSING      │
            └────────────────────────────────┘
                          │
     ┌────────────────────▼────────────────────┐  ～S10
     │  INTERFERENCE FRINGE IMAGE ACQUISITION   │
     │              PROCESSING                  │
     │         (N-th IMAGING CYCLE)             │
     └──────────────────┬───────────────────────┘
                        │                          ～S11
     ┌──────────────────▼───────────────────────┐
     │       SUPER-RESOLUTION PROCESSING         │
     └──────────────────┬───────────────────────┘
                        │                          ～S12
     ┌──────────────────▼───────────────────────┐
     │        RECONSTRUCTION PROCESSING          │
     └──────────────────┬───────────────────────┘
                        │                          ～S13
     ┌──────────────────▼───────────────────────┐
     │   IN-FOCUS POSITION DETECTION PROCESSING  │
     └──────────────────┬───────────────────────┘
                        │                          ～S14
   NO    ◁──────────────▼──────────────────────▷
         │      IN-FOCUS POSITION DETECTION?     │
                        │ YES                      ～S15
     ┌──────────────────▼───────────────────────┐
     │   OPTIMAL RECONSTRUCTED IMAGE OUTPUT      │
     │              PROCESSING                   │
     └──────────────────┬───────────────────────┘
                        │                          ～S16
     ┌──────────────────▼───────────────────────┐
     │   DISPLAY OPTIMAL RECONSTRUCTED IMAGE     │
     └──────────────────┬───────────────────────┘
                        │                          ～S17
         ◁──────────────▼──────────────────────▷  YES
         │        IMAGING OPERATION STOP?        │
                        │ NO                       ～S18
     ┌──────────────────▼───────────────────────┐
     │         INCREMENT (N + 1 → N)             │
     └──────────────────────────────────────────┘

            ┌────────────────────────────────┐
            │              END               │
            └────────────────────────────────┘
```

## FIG. 18

## FIG. 19

# FIG. 20

## INTERNATIONAL SEARCH REPORT

| International application No. |
| --- |
| PCT/JP2021/019618 |

**A. CLASSIFICATION OF SUBJECT MATTER**
Int. Cl. C12M1/34(2006.01)i, G01B9/021(2006.01)i, G01N37/00(2006.01)i
FI: C12M1/34 A, G01B9/021, G01N37/00 101
According to International Patent Classification (IPC) or to both national classification and IPC

**B. FIELDS SEARCHED**

Minimum documentation searched (classification system followed by classification symbols)
Int. Cl. C12M1/34, G01B9/021, G01N37/00

Documentation searched other than minimum documentation to the extent that such documents are included in the fields searched
Published examined utility model applications of Japan      1922-1996
Published unexamined utility model applications of Japan    1971-2021
Registered utility model specifications of Japan            1996-2021
Published registered utility model applications of Japan    1994-2021

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2017-75958 A (IMEC) 20 April 2017, claims, paragraphs [0165], [0173], fig. 2, 6 | 1-10 |
| A | JP 2014-507645 A (THE REGENTS OF THE UNIVERSITY OF CALIFORNIA) 27 March 2014, claims, fig. 1 | 1-10 |
| A | US 2017/0270388 A1 (IMEC VZW) 21 September 2017, claims, fig. 5 | 1-10 |
| A | US 2012/0026509 A1 (CUI, Xiquan et al.) 02 February 2012, claims, paragraph [0053], fig. 6 | 1-10 |

☐ Further documents are listed in the continuation of Box C.          ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 18.06.2021 | 29.06.2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

| International application No. |
| --- |
| PCT/JP2021/019618 |

| Patent Documents referred to in the Report | Publication Date | Patent Family | Publication Date |
| --- | --- | --- | --- |
| JP 2017-75958 A | 20.04.2017 | US 2014/0376816 A1 claims, paragraphs [0177], [0185], fig. 2, 6 EP 2788737 A1 | |
| JP 2014-507645 A | 27.03.2014 | US 2013/0280752 A1 claims, fig. 1 EP 2661603 A2 | |
| US 2017/0270388 A1 | 21.09.2017 | EP 2985719 A1 claims, fig. 5 | |
| US 2012/0026509 A1 | 02.02.2012 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014507645 A
- JP 2017075958 A
- JP 50017134 A
- JP 2001111879 A